# EUROPEAN PATENT APPLICATION

(11) **EP 4 389 874 A1**
(43) Date of publication of application: **26.06.2024**
(21) Application number: 22215949.3
(22) Date of filing: 22.12.2022
(51) Int. Cl.: C12N 1/20, C12N 15/09, C12P 5/02, C12P 7/04, C12P 7/20, C12P 7/24, C12P 7/26

(54) **GENETICALLY MODIFIED MICROORGANISMS FOR THE MANUFACTURE OF COMPOUNDS DERIVED FROM DIHYDROXYACETONE PHOSPHATE**

(71) Applicant: Acies Bio d.o.o., 1000 Ljubljana (SI)
(72) Inventor: KOSEC, Gregor, 1000 Ljubljana (SI); VIRANT, David, 1000 Ljubljana (SI); FUJS, Stefan, 1000 Ljubljana (SI); KRUIS, Aleksander, Johannes, 1000 Ljubljana (SI)
(74) Representative: Zacco GmbH

(57) **Abstract**

The present invention generally relates to the biotechnology engineering, and specifically to genetically engineered microorganisms, e.g. of the genus *Methylobacillus*, which have improved properties making them particularly useful in large scale methanol fermentations. More specifically, the present invention provides a genetically engineered microorganism which is methylotrophic and can generate reducing power by methanol dissimilation, and which microorganism being modified to enable the synthesis of at least one DHAP-derived compound. The present invention further provides a method for producing a biochemical compound using a genetically modified bacterium of the present invention.

## Description

### Technical field of the invention

The present invention relates to the field of biotechnology engineering, and specifically to genetically modified methylotrophic microorganisms which can generate reducing power from methanol and which have been modified to enable the synthesis of compounds derived from dihydroxyacetone phosphate (DHAP). The present invention further relates to a method for producing a biochemical compound derived from DHAP using a genetically modified microorganism.

### Background of the invention

The production of many small biochemical compounds derived from dihydroxyacetone phosphate (DHAP) has been done by fermentation on glucose as the main carbon and energy source. However, compounds such as glycerol, 1,2-propanediol, 1,3-propanediol and 3-hydroxypropionate are difficult to produce from glucose due to their requirements for reducing equivalents in the form of NADH or NADPH. Since no NADH or NADPH is formed on the way to these compounds from glucose, half of the glucose must be used simply to produce the required NADH or NADPH. This reduces yield, effectively wasting half the feedstock. Glycerol is produced from DHAP by reduction via the G3P dehydrogenase (GPD), which consumes an NADH (or NADPH) and then dephosphorylation by glycerol-phosphate phosphorylase (GPP). The DHAP is obtained by cleaving 1,6-FBP into GAP and DHAP. Usually, DHAP is converted into GAP by the TPI enzyme in a reversible reaction and the resulting GAP enters glycolysis and is assimilated. Ideally, all GAP would be converted to DHAP and then glycerol. However, this requires 1 NADH (or NADPH) per glycerol. If produced from glucose, the NADH can only be obtained if GAP enters glycolysis, resulting in 1 pyruvate and 1 glycerol from each molecule of glucose. The pyruvate is in a sense »wasted«. Funneling it back into GAP/DHAP is not feasible, since that would again require NAD(P)H.

Methanol fermentation has the potential to replace chemical production based on fossil sources. To realize this, efficient methylotrophic strains must be developed, i.e. strains consuming one-carbon compounds such as methanol or methane or multi-carbon compounds that contain no carbon-carbon bonds. Several bacteria and yeasts capable of utilizing methanol as the sole source of carbon for growth and product formation are known. Some examples include *Bacillus methanolicus, Methylobacterium extorquens, Methylobacillus glycogenes, Methylobacillus flagellatus, Pichia methanolica* and *Pichia pastoris.* Methylotrophic bacteria can be divided into two broad groups based on their methanol assimilation pathways. The first step is always the conversion of methanol into formaldehyde, catalyzed by a methanol dehydrogenase enzyme. In the first group, formaldehyde reacts with Ribulose-monophosphate (RuMP) to form C6-compounds that are then metabolized further. Members of this group are commonly referred to as "RuMP cycle methylotrophs". The second group, which includes *M*. *extorquens,* assimilate formaldehyde by reacting it with glycine, forming serine in the pathway known as the serine cycle. Both groups of methylotrophic bacteria have been used to produce various biochemicals from methanol. However, the RuMP cycle is more energy efficient than the serine cycle, resulting in faster growth and biomass formation.

Methylotrophic microorganisms can be found in extremely diverse environments, such as oceans, soil, plant rhizosphere and even sewage water. Since they must be capable of surviving these diverse and often harsh environments, methylotrophic bacteria often tend to be quite robust and resistant to different types of stress, all while growing efficiently on minimal nutrients.

Hence there is a need for methylotrophic microorganisms capable of producing DHAP derived compounds from methanol.

### Summary of the invention

The object of the present invention is to overcome certain drawbacks in bioprocesses for the manufacture of DHAP derived compounds. This is achieved by the present inventors who have engineered different methylotrophic microorganisms being modified to enable the synthesis of at least one DHAP-derived compound.

The present inventors unexpectedly and surprisingly found that methylotrophic microorganisms using methanol dissimilation as reducing equivalent source could be modified such that they produce DHAP-derived compounds from methanol, such as glycerol, 1,3-propanediol, 1,2-propanediol and 3-hydroxypropionate. Methanol serves as the sole C source and electron donor (via MeOH dissimilation pathway). Reducing equivalents are obtained via glucose-6-phosphate 1-dehydrogenase and glucose-6-phosphate 1-dehydrogenase, via formaldehyde dehydrogenase and formate dehydrogenase or via Methylene-THMPT pathway. The methylotrophic microorganisms was *Methylobacillus flagellatus.*

The present inventors further surprisingly found that forced, growth-coupled DHAP production from methanol can be done in methylotrophic microorganisms, such as *Methylobacillus spp.* The forced DHAP production is e.g. by deletion of triosephosphate isomerase (TPI), controlling of flux towards biomass/product by regulating expression ratio between phosphofructokinase (pfk) phosphogluconate dehydratase (EDD) and by reduced EDD and increased PFK expression.

Based on the foregoing findings, the present invention provides in a first aspect a genetically engineered microorganism which is methylotrophic and can generate reducing power by methanol dissimilation, and said microorganism being modified to enable the synthesis of at least one DHAP-derived compound.

The present invention further provides a method to produce a biochemical compound derived from DHAP comprising cultivating a genetically engineered microorganism of the present invention under suitable culture conditions in a culture medium comprising a reduced one-carbon compound, such as methanol, or a multi-carbon compound that contains no carbon-carbon bonds, such as dimethylamine or methylamine.

The present invention may be summarized by the following items:
1. A genetically engineered microorganism which is methylotrophic and can generate reducing power by methanol dissimilation, and said microorganism being modified to enable the synthesis of at least one DHAP-derived compound.
2. The genetically engineered microorganism according to item 1, which can metabolize methanol as the main carbon source and main electron donor via the methanol dissimilation pathway.
3. The genetically engineered microorganism according to any one of items 1-2, wherein reducing equivalents are obtained via NAD- and or NADP dependent glucose-6-phosphate 1-dehydrogenase and NAD and/or NADP- dependent 6-phosphogluconate dehydrogenase.
4. The genetically engineered microorganism according to any one of items 1-2, wherein reducing equivalents are obtained via formaldehyde dehydrogenase and/or formate dehydrogenase.
5. The genetically engineered microorganism according to any one of items 1-2, wherein reducing equivalents are obtained via the methylene-THMPT pathway.
6. The genetically engineered microorganism according to any one of items 1-5, comprising the ribulose monophosphate (RuMP) methanol assimilation pathway.
7. The genetically engineered microorganism according to item 6, wherein said genetically engineered microorganism has the EDA variant of RuMP.
8. The genetically engineered microorganism according to any one of items 1-7, wherein said DHAP-derived compound has three carbons.
9. The genetically engineered microorganism according to any one of items 1-8, wherein said DHAP-derived compound has the structure of Formula I: 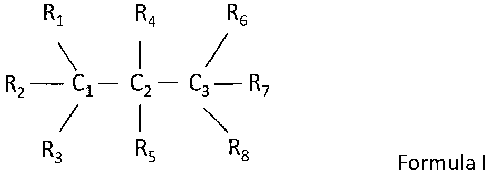
   wherein R₁, R₂, R₃, R₄, R₅, R₆ and R₇ are independently selected from hydrogen, hydroxyl, and pairwise on each of carbons C₁ to C₃ may form an oxo group, and
   wherein R₈ is selected from hydrogen, hydroxyl and phosphate.
10. The genetically engineered microorganism according to item 9, wherein R₁ and R₂ together form an oxo group to form the structure of Formula II : 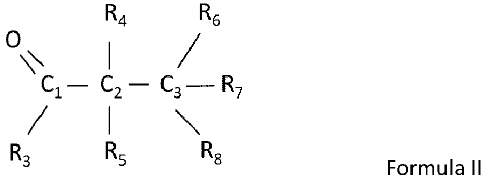
11. The genetically engineered microorganism according to any one of items 9-10, wherein R₆ and R₇ together form an oxo group to form the structure of Formula IIIa or Formula IIIb : 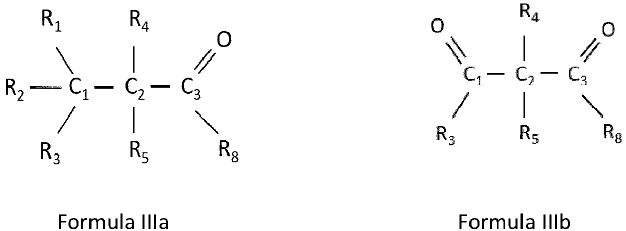
12. The genetically engineered microorganism according to any one of items 1-9, wherein said DHAP-derived compound is an alcohol.
13. The genetically engineered microorganism according to any one of items 1-12, wherein said DHAP-derived compound is an aldehyde.
14. The genetically engineered microorganism according to any one of items 1-12, wherein said DHAP-derived compound is a carboxylic acid.
15. The genetically engineered microorganism according to any one of items 1-14, wherein said DHAP-derived compound is selected from the group consisting of glycerol, 1,3-propanediol, 1,2-propanediol, methylglyoxal (MGO), 2-hydroxypropanal (lactaldehyde), 3-hydroxypropanal (reuterin), hydroxyacetone (acetol), dihydroxyacetone (glycerone), 3-hydroxypropionate, lactate, malonate, glycerone, acetone and dihydroxyacetone phosphate.
16. The genetically engineered microorganism according to any one of items 1-9 and 12, wherein said DHAP-derived compound is glycerol.
17. The genetically engineered microorganism according to any one of items 1-16, which expresses at least one NADH- or NADPH-dependent glycerol-phosphate dehydrogenase and at least one glycerol phosphatase.
18. The genetically engineered microorganism according to item 17, which expresses at least one NADH- or NADPH-dependent glycerol-phosphate dehydrogenase selected from the group consisting of SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125 and functionally equivalent enzymes having at least 95% sequence identity to any of SEQ ID NO: 116 to SEQ ID NO: 125.
19. The genetically engineered microorganism according to item 18, which expresses at least one NADH- or NADPH-dependent glycerol-phosphate dehydrogenase selected from the group consisting of SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124 and SEQ ID NO: 125.
20. The genetically engineered microorganism according to any one of items 1-9, 12-13 and 17-19, wherein said DHAP-derived compound is 1,3-propanediol.
21. The genetically engineered microorganism according to any one of items 1-9, 12-13 and 17-19, wherein said DHAP-derived compound is 3-hydroxypropionate.
22. The genetically engineered microorganism according to any one of items 1-9, 12 and 17-19, wherein said DHAP-derived compound is 3-hydroxypropanal (reuterin).
23. The genetically engineered microorganism according to any one of items 1-11 and 13-15, wherein said DHAP-derived compound is methylglyoxal (MGO).
24. The genetically engineered microorganism according to any of items 1-16 and 23, which expresses at least one methylglyoxal synthase.
25. The genetically engineered microorganism according to item 24, which expresses at least one methylglyoxal synthase selected from the group consisting of SEQ ID NO: 141, SEQ ID NO: 142, SEQ ID NO: 143, SEQ ID NO: 144, SEQ ID NO: 145, SEQ ID NO: 146 and functionally equivalent enzymes having at least 95% sequence identity to any of SEQ ID NO: 141 to SEQ ID NO: 146.
26. The genetically engineered microorganism according to item 25, which expresses at least one methylglyoxal synthase selected from the group consisting of SEQ ID NO: 141, SEQ ID NO: 142, SEQ ID NO: 143, SEQ ID NO: 144, SEQ ID NO: 145 and SEQ ID NO: 146.
27. The genetically engineered microorganism according to any one of items 1-12, 15 and 24-26, wherein said DHAP-derived compound is 2-hydroxypropanal.
28. The genetically engineered microorganism according to any one of items 1-12, 15, and 24-26 wherein said DHAP-derived compound is 1,2-propanediol.
29. The genetically engineered microorganism according to any one of items 1-13, wherein said DHAP-derived compound is 2-hydroxypropanal (lactaldehyde).
30. The genetically engineered microorganism according to item 29, which expresses at least one methylglyoxal synthase and at least one lactaldehyde dehydrogenase.
31. The genetically engineered microorganism according to item 30, which expresses at least one lactaldehyde dehydrogenase selected from the group consisting of SEQ ID NO: 157, SEQ ID NO: 158, SEQ ID NO: 159, SEQ ID NO: 160, SEQ ID NO: 161 and functionally equivalent enzymes having at least 95% sequence identity to any of SEQ ID NO: 157 to SEQ ID NO: 161.
32. The genetically engineered microorganism according to item 31, which expresses at least one lactaldehyde dehydrogenase selected from the group consisting of SEQ ID NO: 157, SEQ ID NO: 158, SEQ ID NO: 159, SEQ ID NO: 160 and SEQ ID NO: 161.
33. The genetically engineered microorganism according to any one of items 1-9, 12, 15 and 23-26, wherein said DHAP-derived compound is hydroxyacetone (acetol).
34. The genetically engineered microorganism according to any of items 1-9, 15 and 33, which expresses at least one methylglyoxal synthase and at least one methylglyoxal-specific alcohol dehydrogenase.
35. The genetically engineered microorganism according to item 34, which expresses at least one methylglyoxal-specific alcohol dehydrogenase selected from the group consisting of SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156 and functionally equivalent enzymes having at least 95% sequence identity to any of SEQ ID NO: 147 to SEQ ID NO: 156.
36. The genetically engineered microorganism according to item 35, which expresses at least one methylglyoxal synthase selected from the group consisting of SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155 and SEQ ID NO: 156.
37. The genetically engineered microorganism according to any one of items 1-12, wherein said DHAP-derived compound is dihydroxyacetone (glycerone).
38. The genetically engineered microorganism according to item 37, which expresses dihydroxyacetone reductase.
39. The genetically engineered microorganism according to item 38, wherein said dihydroxyacetone reductase is selected from the group consisting of SEQ ID NO: 137, SEQ ID NO: 138, SEQ ID NO: 139, SEQ ID NO: 140, and functionally equivalent enzymes having at least 95% sequence identity to any of SEQ ID NO: 137 to SEQ ID NO: 140.
40. The genetically engineered microorganism according to item 39, wherein said dihydroxyacetone reductase is selected from the group consisting of SEQ ID NO: 137, SEQ ID NO: 138, SEQ ID NO: 139 and SEQ ID NO: 140.
41. The genetically engineered microorganism according to item 37, which expresses glycerol dehydrogenase.
42. The genetically engineered microorganism according to item 41, wherein said dihydroxyacetone reductase is selected from the group consisting of SEQ ID NO: 133, SEQ ID NO: 134, and functionally equivalent enzymes having at least 95% sequence identity to any of SEQ ID NO: 133 or SEQ ID NO: 134.
43. The genetically engineered microorganism according to item 42, wherein said dihydroxyacetone reductase is SEQ ID NO: 133 or SEQ ID NO: 134.
44. The genetically engineered microorganism according to any one of items 1-13, wherein said DHAP-derived compound is lactate.
45. The genetically engineered microorganism according to item 44, which expresses at least one methylglyoxal synthase and at least one lactate dehydratase.
46. The genetically engineered microorganism according to item 45, which expresses at least one lactate dehydratase selected from the group consisting of SEQ ID NO: 170, SEQ ID NO: 171, SEQ ID NO: 172, SEQ ID NO: 173 and functionally equivalent enzymes having at least 95% sequence identity to any of SEQ ID NO: 170 to SEQ ID NO: 173.
47. The genetically engineered microorganism according to item 45, which expresses at least one methylglyoxal synthase selected from the group consisting of SEQ ID NO: 170, SEQ ID NO: 171, SEQ ID NO: 172 and SEQ ID NO:173.
48. The genetically engineered microorganism according to any one of items 1-47, wherein there is absence of expression of triosephosphate isomerase (TPI).
49. The genetically engineered microorganism according to items 48, wherein the absence of expression of triosephosphate isomerase (TPI) is due to deletion of all tpi gene(s) or due to mutations in all the tpi gene(s) or their regulatory sequences.
50. The genetically engineered microorganism according to any of items 1-49, wherein the expression of phosphogluconate dehydratase (EDD) is lowered and the expression of phosphofructokinase (pfk) is increased.
51. The genetically engineered microorganism according to any of items 48-50, which can express a heterologous phosphofructokinase.
52. The genetically engineered microorganism according to any one of items 1-51, which is a bacterium.
53. The genetically engineered microorganism according to item 52, wherein said bacterium is a *Methylobaccilus sp.*
54. The genetically engineered microorganism according to item 53, wherein said bacterium is selected from the group consisting of *Methylobacillus flagellatus, Methylobacillus glycogenes, Methylobacillus pratensis, Methylobacillus rhizosphaerae, Methylobacillus gramineus, Methylobacillus arboreus, Methylobacillus caricics* and *Methylobacillus methilovorans.*
55. The genetically engineered microorganism according to any one of items 53-54, wherein said bacterium is *Methylobacillus flagellatus or Methylobacillus glycogenes.*
56. The genetically engineered microorganism according to item 55, wherein said bacterium is *Methylobacillus flagellatus.*
57. The genetically engineered microorganism according to item 55, wherein said bacterium is *Methylobacillus glycogenes.*
58. A method for the production of a biochemical compound derived from DHAP comprising cultivating a microorganism according to any one of items 1-57 under suitable culture conditions in a culture medium comprising a reduced one-carbon compound, such as methanol, or a multi-carbon compound that contains no carbon-carbon bonds, such as dimethylamine or methylamine.
59. The method according to item 58, wherein the culture medium comprises methanol.
60. The method according to item 58 or 59, wherein the cultivation is performed in a bioreactor.

### Brief description of the figures

**Figure 1****:** Sources of reducing equivalents in methanol dissimilation.
**Figure 2****:** Utilization of reducing equivalents from methanol dissimilation for DHAP-derived product synthesis in an EDA-RuMP methylotroph.

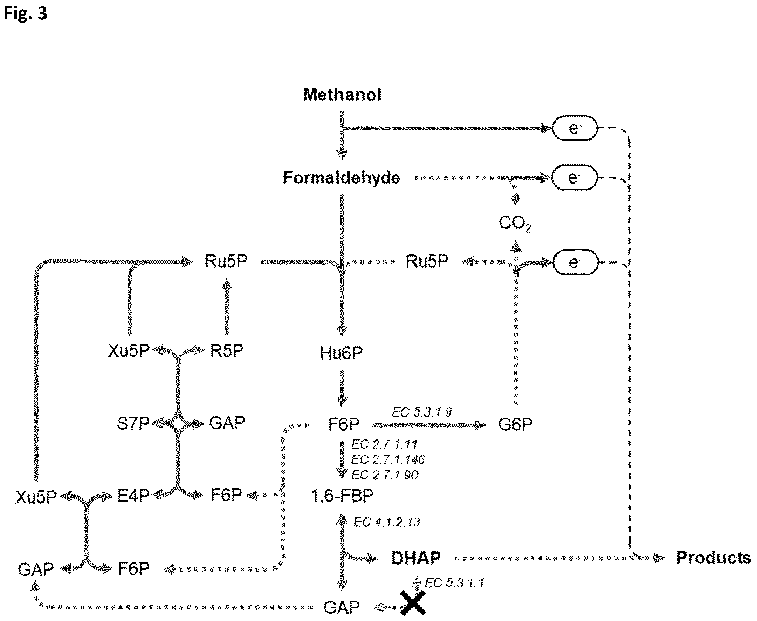

**Figure 3****:** Utilization of reducing equivalents from methanol dissimilation for DHAP-derived product synthesis in a modified PFK-RuMP methylotroph.
**Figure 4****:** DHAP-derived product pathways.
**Figure 5****:** Production of glycerol from methanol in shake flasks.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory).

### Metabolic principles of the methylotrophic microorganisms of the invention

Normally DHAP is prepared from glycerol, such as from bio-waste containing glycerol. Here it is proposed to make DHAP derived compounds e.g., glycerol from methanol. Several variants of the RuMP methanol assimilation pathway exist. In the more common FBA variant, F6P is converted to 1,6-FBP, which is then cleaved into GAP and DHAP. The DHAP is then converted to GAP by the enyzme triosephosphate isomerase (TPI). One of the GAP molecules re-enters the RuMP cycle while the other enters glycolysis and is eventually converted to pyruvate, generating NADH and 2 ATP. *Methylobacillus OCB480* on the other hand assimilates methanol via the so called EDA variant of the RuMP pathway. Here, F6P is converted to 2,3 KDPG through a series of reactions, generating NAD(P)H, but no ATP. The 2,3 KDPG is then cleaved into a pyruvate and a GAP. The GAP again re-enters the cycle, while the pyruvate can be used for other things (biomass). The pyruvate can also enter glycolysis in the opposite direction, consuming ATP in order to be converted into GAP.

In any case, this is not ideal for glycerol production. The GAP obtained must re-enter the RuMP cycle and converting pyruvate into DHAP is costly. However, the addition of a phosphofructokinase (PFK) enzyme allows for conversion of F6P to 1,6-FBP. This basically ads the FBA pathway on top of the native EDA pathway and allows the strain to produce GAP and DHAP directly. Additionally, deletion of the TPI would mean that GAP and DHAP cannot be interconverted. This would mean that every DHAP produced could only be converted to gylcerol or other products, while the GAP re-enters the RuMP cycle to maintain metabollite balance. This requires additional reducing power in the form of NADH or NADPH, but this is obtained upstream from MeOH and formate. **Since every** **molecule of glycerol requires 1 NAD(P)H and each molecule of MeOH produces 2 NAD(P)H when fully converted to CO2, this would mean that only 1 C-mole would be wasted for each 6 C-moles of glycerol (or similar)**, **wasting roughly 14% of carbon. This is significantly better than on glucose, where 50% is »wasted«.** All of this is without an NADH-dependent methanol dehydrogenase (MDH). If an NADH-dependent MDH is used, no carbon is be wasted - in fact, additional CO2 could be fixed.

The native EDA pathway is left as is to be used for biomass formation, and flux of carbon towards DHAP and products may be driven by overexpression of PFK. Alternatively, the native EDA pathway can be downtregulated by reducing expression of the EDD gene (phosphogluconate dehydratase).

### DHAP-derived compounds.

There are quite a number of DHAP derived compounds and some of them are described by Formula I: 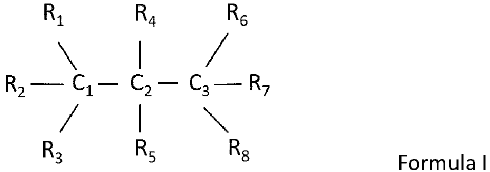
wherein R₁, R₂, R₃, R₄, R₅, R₆ and R₇ are independently selected from hydrogen, hydroxyl, and pairwise on each of carbons C₁ to C₃ may form an oxo group, and
wherein R₈ is selected from hydrogen, hydroxyl, and phosphate.

Table 1 lists the specific substituents R₁ to R₈ which are found in the DHAP-derived compounds glycerol, 1,2-propanediol, 1,3-propanediol, methylglyoxal, 2-hydroxypropanal, 3-hydroxypropanal, hydroxyacetone, dihydroxyacetone, 3-hydroxypropionate, lactate, malonate, glycerone, acetone and dihydroxyacetone phosphate.

**Table 1. Some DHAP-derived compounds having the structure of Formula I.**

| **Compound** | **R₁** | **R₂** | **R₃** | **R₄** | **R₅** | **R₆** | **R₇** | **R₈** |
|---|---|---|---|---|---|---|---|---|
| Glycerol | H | OH | H | OH | H | H | OH | H |
| 1,2-Propanediol | H | H | H | OH | H | H | OH | H |
| 1,3-Propanediol | H | OH | H | H | H | H | OH | H |
| Methylglyoxal | H | H | H | O | / | O | H | / |
| 2-hydroxypropanal | H | H | H | OH | H | O | H | / |
| 3-hydroxypropanal | / | O | H | H | H | H | OH | H |
| Hydroxyacetone | H | H | H | O | / | H | OH | H |
| Dihydroxyacetone | H | OH | H | O | / | H | OH | H |
| 3-Hydroxypropionate | H | OH | H | H | H | O | OH | / |
| Lactate | H | H | H | OH | H | O | OH | / |
| Malonate | / | O | OH | H | H | O | OH | / |
| Glycerone | H | OH | H | OH | H | H | OH | H |
| Acetone | H | H | H | O | / | H | H | H |
| Dihydroxyacetone phosphate | H | OH | H | O | / | H | H | PO₄ |

| | | | | | | | | |
|---|---|---|---|---|---|---|---|---|
| "O" means an oxo group formed together with the substituent on the same carbon which is marked "/". | | | | | | | | |

In some embodiments, the DHAP-derived compound is an alcohol.

In some other embodiments, the DHAP-derived compound is an aldehyde.

In some other embodiments, the DHAP-derived compound is a carboxylic acid.

In some other embodiments, the DHAP-derived compound is selected from the group consisting of glycerol, 1,3-propanediol, 1,2-propanediol, methylglyoxal (MGO), 2-hydroxypropanal (lactaldehyde), 3-hydroxypropanal (reuterin), hydroxyacetone (acetol), dihydroxyacetone (glycerone), 3-hydroxypropionate, lactate, malonate, glycerone, acetone and dihydroxyacetone phosphate.

In some other embodiments, the DHAP-derived compound is glycerol.

### Microorganisms of the invention

As indicated above, the present invention is based on the unexpected and surprising finding that certain drawbacks in bioprocesses for the manufacture of DHAP derived compounds can be overcome by modifying methylotrophic microorganisms to enable the synthesis at least one DHAP-derived compound from methanol.

The present invention thus provides in a first aspect a genetically engineered microorganism which is methylotrophic and can generate reducing power by methanol dissimilation, and said microorganism being modified to enable the synthesis of at least one DHAP-derived compound.

Different methylotrophic microorganisms can serve as host cells for this modification. Examples of methylotrophic microorganisms are found among both bacteria and yeasts, e.g. *Methylobacillus sp. , Bacillus sp.* and *Pichia sp.*

More specifically, the present invention provides a genetically engineered microorganism such as a bacterium of the genus *Methylobacillus* which has been modified to enable the synthesis of at least one DHAP-derived compound.

A bacterium in accordance with the present invention can be produced from any suitable bacterium which can generate reducing power by methanol dissimilation, e.g. of the genus *Methylobacillus. Methylobacillus* is a genus of Gram-negative methylotrophic bacteria.

According to some embodiments, the bacterium of the present invention is selected from *Methylobacillus flagellatus, Methylobacillus glycogenes, Methylobacillus pratensis and Methylobacillus rhizosphaerae.*

According to some embodiments, the bacterium of the present invention is selected from *Methylobacillus flagellatus* and *Methylobacillus glycogenes.*

According to some embodiments, the bacterium of the present invention is *Methylobacillus flagellatus.*

According to some embodiments, the bacterium of the present invention is *Methylobacillus glycogenes.*

According to some embodiments, the genetically engineered microorganism expresses at least one NADH- or NADPH-dependent glycerol-phosphate dehydrogenase and at least one glycerol phosphatase. Such a genetically engineered microorganism is useful for the synthesis of 1,3-propanediol, 3-hydroxypropionate, 3-hydroxypropanal (reuterin).

According to some embodiments, the genetically engineered microorganism expresses at least one NADH- or NADPH-dependent glycerol-phosphate dehydrogenase selected from the group consisting of SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125 and functionally equivalent enzymes having at least 95% sequence identity to any of SEQ ID NO: 116 to SEQ ID NO: 125.

According to some embodiments, the genetically engineered microorganism expresses at least one NADH- or NADPH-dependent glycerol-phosphate dehydrogenase selected from the group consisting of SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124 and SEQ ID NO: 125.

According to some embodiments, the genetically engineered microorganism expresses at least one methylglyoxal synthase. Such a genetically engineered microorganism is useful for the synthesis of methylglyoxal, 2-hydroxypropanal, 1,2-propanediol.

According to some embodiments, the genetically engineered microorganism expresses at least one methylglyoxal synthase selected from the group consisting of SEQ ID NO: 141, SEQ ID NO: 142, SEQ ID NO: 143, SEQ ID NO: 144, SEQ ID NO: 145, SEQ ID NO: 146 and functionally equivalent enzymes having at least 95% sequence identity to any of SEQ ID NO: 141 to SEQ ID NO: 146.

According to some embodiments, the genetically engineered microorganism expresses at least one methylglyoxal synthase selected from the group consisting of SEQ ID NO: 141, SEQ ID NO: 142, SEQ ID NO: 143, SEQ ID NO: 144, SEQ ID NO: 145 and SEQ ID NO: 146.

According to some embodiments, the genetically engineered microorganism expresses at least one methylglyoxal synthase and at least one lactaldehyde dehydrogenase. Such a genetically engineered microorganism is useful for the synthesis of 2-hydroxypropanal (lactaldehyde).

According to some embodiments, the genetically engineered microorganism expresses at least one lactaldehyde dehydrogenase selected from the group consisting of SEQ ID NO: 157, SEQ ID NO: 158, SEQ ID NO: 159, SEQ ID NO: 160, SEQ ID NO: 161 and functionally equivalent enzymes having at least 95% sequence identity to any of SEQ ID NO: 157 to SEQ ID NO: 161.

According to some embodiments, the genetically engineered microorganism expresses at least one lactaldehyde dehydrogenase selected from the group consisting of SEQ ID NO: 157, SEQ ID NO: 158, SEQ ID NO: 159, SEQ ID NO: 160 and SEQ ID NO: 161.

According to some embodiments, the genetically engineered microorganism expresses at least one methylglyoxal synthase and at least one methylglyoxal-specific alcohol dehydrogenase.

According to some embodiments, the genetically engineered microorganism expresses at least one methylglyoxal-specific alcohol dehydrogenase selected from the group consisting of SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156 and functionally equivalent enzymes having at least 95% sequence identity to any of SEQ ID NO: 147 to SEQ ID NO: 156.

According to some embodiments, the genetically engineered microorganism expresses at least one methylglyoxal synthase selected from the group consisting of SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155 and SEQ ID NO: 156.

According to some embodiments, the genetically engineered microorganism expresses dihydroxyacetone reductase. Such a genetically engineered microorganism is useful for the synthesis of dihydroxyacetone (glycerone).

According to some embodiments, the genetically engineered microorganism expresses at least one dihydroxyacetone reductase which is selected from the group consisting of SEQ ID NO: 137, SEQ ID NO: 138, SEQ ID NO: 139, SEQ ID NO: 140, and functionally equivalent enzymes having at least 95% sequence identity to any of SEQ ID NO: 137 to SEQ ID NO: 140.

According to some embodiments, the genetically engineered microorganism expresses at least one dihydroxyacetone reductase which is selected from the group consisting of SEQ ID NO: 137, SEQ ID NO: 138, SEQ ID NO: 139 and SEQ ID NO: 140.

According to some embodiments, the genetically engineered microorganism expresses glycerol dehydrogenase.

According to some embodiments, the genetically engineered microorganism expresses dihydroxyacetone reductase which is selected from the group consisting of SEQ ID NO: 133, SEQ ID NO: 134, and functionally equivalent enzymes having at least 95% sequence identity to any of SEQ ID NO: 133 or SEQ ID NO: 134.

According to some embodiments, the genetically engineered microorganism which expresses dihydroxyacetone reductase is SEQ ID NO: 133 or SEQ ID NO: 134.

According to some embodiments, the genetically engineered microorganism expresses at least one methylglyoxal synthase and at least one lactate dehydratase. Such a genetically engineered microorganism is useful for the synthesis of lactate (lactic acid).

According to some embodiments, the genetically engineered microorganism expresses at least one lactate dehydratase selected from the group consisting of SEQ ID NO: 170, SEQ ID NO: 171, SEQ ID NO: 172, SEQ ID NO: 173 and functionally equivalent enzymes having at least 95% sequence identity to any of SEQ ID NO: 170 to SEQ ID NO: 173.

According to some embodiments, the genetically engineered microorganism expresses at least one methylglyoxal synthase selected from the group consisting of SEQ ID NO: 170, SEQ ID NO: 171, SEQ ID NO: 172 and SEQ ID NO:173.

According to some embodiments, the genetically engineered microorganism which has absence of expression of triosephosphate isomerase (TPI).

According to some embodiments, the genetically engineered microorganism which has absence of expression of triosephosphate isomerase (TPI), this has been obtained by deletion of all tpi gene(s) or has been obtained by mutations in all the tpi gene(s) or their regulatory sequences.

According to some embodiments, the genetically engineered microorganism has an expression of phosphogluconate dehydratase (EDD) which is lowered, and the expression of phosphofructokinase (pfk) is increased. This lowered and increased expression, respectively, are as compared to the microorganism without said modification.

According to some embodiments, the genetically engineered microorganism has an expression of phosphogluconate dehydratase (EDD) which is lowered by at least 50%, such as by at least 60%, at least 65%, at least 70%, at least 75%, at least 80%, at least 85%, at least 90% or at least 95% compared to the otherwise identical bacterium, and the genetically engineered microorganism has an expression of phosphofructokinase (pfk) which is increased by at least 10%, such as by at least 20%, at least 30%, at least 50%, at least 100% or at least 200% compared to an otherwise identical bacterium.

According to some embodiments, the expression of phosphogluconate dehydratase (EDD) in the genetically engineered microorganism is decreased by way of inhibition.

Inhibition of the expression of phosphogluconate dehydratase may be achieved by any suitable means known in the art. For example, the expression may be inhibited by gene silencing techniques involving the use of inhibitory nucleic acid molecules, such as antisense oligonucleotides, ribozymes or interfering RNA (RNAi) molecules, such as microRNA (miRNA), small interfering RNA (siRNA) or short hairpin RNA (shRNA).

According to some embodiments, the expression of phosphogluconate dehydratase (EDD) in the genetically engineered microorganism is decreased (e.g., inhibited) by transcriptional and/or translational repression of the endogenous gene encoding said phosphogluconate dehydratase.

According to some embodiments, the expression of phosphogluconate dehydratase (EDD) in the genetically engineered microorganism is inhibited by introducing or expressing in the microorganism an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypeptide in question. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide in question.

According to some embodiments, the inhibitory nucleic acid is an antisense oligonucleotide. Such antisense oligonucleotide is a nucleic acid molecule (either DNA or RNA) which specifically hybridizes (e.g. binds) under cellular conditions with the cellular mRNA and/or genomic DNA encoding the enzyme in question.

According to some embodiments, the inhibitory nucleic acid molecule is a ribozyme, such as a hammerhead ribozyme. A ribozyme molecule is designed to catalytically cleave the mRNA transcript to prevent translation of the polypeptide in question.

According to some embodiments, the inhibitory nucleic acid molecule is an interfering RNA (RNAi) molecule. RNA interference is a biological process in which RNA molecules inhibit expression, typically causing the destruction of specific mRNA. Exemplary types of RNAi molecules include microRNA (miRNA), small interfering RNA (siRNA) and short hairpin RNA (shRNA). According to some embodiments, the RNAi molecule is a miRNA. According to some embodiments, the RNAi molecule is a siRNA. According to some embodiments, the RNAi molecule is a shRNA.

According to some embodiments, the genetically engineered microorganism has a RuMP methanol assimilation pathway.

According to some embodiments, the genetically engineered microorganism has the EDA variant of RuMP.

According to some embodiments, the genetically engineered microorganism expresses a heterologous phosphofructokinase.

### Listing of polypeptide sequences

The polypeptides in the sequence listing are described in the aspects, embodiments and examples of the present invention. Many of the listed polypeptides are found in microorganisms and the specific microorganism, the polypeptide name and the encoding gene may be found in e.g. UNIPROT or similar databses of polypeptides.

### Method of the invention

The present invention also provides methods for producing a biochemical compound comprising cultivating a genetically engineered microorganism according to the invention under suitable culture conditions in a culture medium comprising a reduced one-carbon compound, such as methanol, or a multi-carbon compound that contain no carbon-carbon bonds, such as dimethylamine.

The culture medium employed may be any conventional medium suitable for culturing a microorganism in question, and may be composed according to the principles known in the art. The medium will usually contain all nutrients necessary for the growth and survival of the respective microorganism, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing microorganisms such as bacterial and yeast cells, such as *E. coli* cells, including minimal media and rich media such as Luria Broth (LB), M9 media, M17 media, SA media, MOPS media, Terrific Broth, YT, and others.

The carbon source may be any suitable carbon substrate known in the art, and in particularly any carbon substrate commonly used in the cultivation of methylotrophic bacteria and/or fermentation. A carbon source of particular interest is a reduced one-carbon compound, such as methanol or methylamine, or a multi-carbon compound that contains no carbon-carbon bonds, such as dimethylamine. Thus, according to some embodiments, the culture medium comprises methanol as a carbon source. The concentration of methanol in the culture medium may generally be in the range from about 0,5% w/v to about 4 % w/v, such as from about 2% w/v to about 4 % w/v. According to some embodiments, the concentration of methanol in the culture medium is in the range from about 2,5% w/v to about 3,5% w/v.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used.

The cultivation can be preferably performed under aerobic conditions, such as by a shaking culture, by a stirring culture or in a bioreactor with aeration, at a temperature suitable for the microorganisms in question, such as of about 20 to about 45 °C, such as about 30 to 38 °C, preferably at about 37°C. The pH of the culture is usually above 5, such as in a range from about 6 to about 8, preferably from about 6.5 to about 7.5, more preferably from about 6.8 to about 7.2.

The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as cells can be removed from the culture medium by centrifugation or membrane filtration. The biochemical compound can be collected by conventional method for isolation and purification chemical compounds from a medium. Well-known purification procedures include, but are not limited to, centrifugation or filtration, precipitation, ion exchange, chromatographic methods such as e.g. ion exchange chromatography or gel filtration chromatography, and crystallization methods. The method may further comprise collecting the biochemical compound from the culture medium.

The biochemical compound produced by the method of the present invention may be any desired compound obtained through the cultivation of a microorganism of the present invention. Non-limiting examples include organic acids, organic aldehydes, organic alcohols as also exemplified by Formula I and described above.

The present invention thus provides a biochemical compound obtainable by a method as detailed herein.

### Certain other abbreviations and definitions

### Abbreviations:

- 1,2-PDO: 1,2-hydroxyproprionate
- 1,3-PDO: 1,3-hydroxyproprionate
- 2,3-KDPG: 2-Keto-3-deoxy-6-phosphogluconate
- 3-HP: 3-hydroxypropionate
- DHAP: Dihydroxyacetone phosphate
- EDA: Gene for Entner-Doudoroff aldolase (2-keto-3-deoxy-6-phosphogluconate aldolase)
- EDD: Gene for phosphogluconate dehydratase
- GAP: Glyceraldehyde 3-phosphate
- MDH: Methanol dehydrogenase
- PFK: Phosphofructokinase
- RuMP: Ribulose-monophosphate
- TPI: Triosephosphate isomerase

### Definitions:

"Polypeptide" and "protein" are used interchangeably herein to denote a polymer of at least two amino acids covalently linked by an amide bond, regardless of length or post- translational modification (e.g., glycosylation, phosphorylation, lipidation, myristilation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a host cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genes that are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it is present or which is found in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression" of a gene it is meant that the amount of the transcription product, respectively the amount of the polypeptide (e.g., enzyme) encoded by said gene produced by the modified bacterium is decreased compared to an otherwise identical bacterium that does not carry said modification. More particularly, by "decreased expression" of a gene it is meant that the amount of the transcription product, respectively the amount of the polypeptide (e.g., enzyme) encoded by said gene produced by the modified bacterium is decreased by at least 10%, such as at least 20%, at least 30%, at least 40%, at least 50% at least 60%, at least 70%, at least 80%, at least 90% or at least 100%, compared to an otherwise identical bacterium that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

Expression of a gene can be decreased by introducing a mutation into the gene in the genome of the bacterium so that the intracellular activity of the polypeptide encoded by the gene is decreased as compared to an otherwise identical bacterium that does not carry said mutation. Mutations which result in a decreased expression of the gene include the replacement of one nucleotide or more to cause an amino acid substitution in the polypeptide encoded by the gene (missense mutation), introduction of a stop codon (nonsense mutation), deletion or insertion of nucleotides to cause a frame shift, insertion of a drug-resistance gene, or deletion of a part of the gene or the entire gene (Qiu and Goodman, 1997; Kwon et al., 2000). Expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The lambda-red mediated gene replacement is a particularly suitable method to inactive one or more genes as described herein.

"Inactivating", "inactivation" and "inactivated", when used in the context of a gene or gene cluster, means that the gene or gene cluster in question no longer expresses a functional protein. It is possible that the modified DNA region is unable to naturally express the gene or gene cluster due to the deletion of a part of or the entire sequence of the gene or gene cluster, the shifting of the reading frame of the gene or gene cluster, the introduction of missense/nonsense mutation(s), or the modification of the regulatory region of the gene or gene cluster, including sequences controlling gene expression, such as a promoter, enhancer, attenuator, ribosome- binding site, etc. Preferably, a gene or gene cluster of interest is inactivated by deletion of a part of or the entire sequence of the gene or gene cluster, such as by gene replacement. Inactivation may also be accomplished by introducing or expressing a rare-cutting endonuclease able to selectively inactivate by DNA cleavage, preferably by double-strand break, the gene or gene cluster of interest. A "rare-cutting endonuclease" within the context of the present invention includes transcription activator-like effector (TALE) nucleases, meganucleases, zinc-finger nucleases (ZFN), and RNA-guided endonucleases.

The presence or absence of a gene or gene cluster in the genome of a bacterium can be detected by well-known methods, including PCR, Southern blotting, and the like. In addition, the level of gene expression can be estimated by measuring the amount of mRNA transcribed from the gene or gene cluster using various well-known methods, including Northern blotting, quantitative RT-PCR, and the like. The amount of the polypeptide encoded by the gene or gene cluster can be measured by well-known methods, including SDS-PAGE followed by an immunoblotting assay (Western blotting analysis), and the like.

As used herein, "decreased", "decreasing" or "decrease of" expression of a polypeptide (such as an enzyme as described herein) means that the expression of said polypeptide in a modified bacterium is reduced compared to the expression of said polypeptide in an otherwise identical bacterium that does not carry said modification (control). The expression of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). More particularly, "decreased", "decreasing" or "decrease of" expression of a polypeptide means that the amount of the polypeptide in the modified bacterium is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical bacterium that does not carry said modification (control). The expression or amount of a polypeptide in a bacterium can be determined by any suitable means know in the art, including techniques such as ELISA, Immunohistochemistry, Western Blotting or Flow Cytometry.

As used herein, "abolished" expression of a polypeptide (such as an enzyme as described herein) means that the expression of said polypeptide in a modified bacterium is not detectable compared to the expression of said polypeptide in an otherwise identical bacterium that does not carry said modification (control).

As used herein, "decreased", "decreasing" or "decrease of" activity of a polypeptide (such as an enzyme as described herein) means that the catalytic activity of said polypeptide in a modified microorganism is reduced compared to the catalytic activity of said polypeptide in an otherwise identical microorganism that does not carry said modification (control). The activity of a polypeptide in a modified microorganism may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical microorganism that does not carry said modification (control). The activity of a polypeptide in a microorganism can be determined by any suitable protein and enzyme activity assay.

"Expression" includes any step involved in the production of a polypeptide (e.g., encoded enzyme) including, but not limited to, transcription, post-transcriptional modification, translation, post-translational modification, and secretion.

As used herein, "regulatory region" of a gene or gene cluster refers to a nucleic acid sequence that affect the expression of a coding sequence. Regulatory regions are known in the art and include, but are not limited to, promoters, enhancers, transcription terminators, polyadenylation sites, matrix attachment regions and/or other elements that regulate expression of a coding sequence.

"Substitution" or "substituted" refers to modification of the polypeptide by replacing one amino acid residue with another, for instance the replacement of a Serine residue with a Glycine or Alanine residue in a polypeptide sequence is an amino acid substitution. When used with reference to a polynucleotide, "substitution" or "substituted" refers to modification of the polynucleotide by replacing one nucleotide with another, for instance the replacement of a cytosine with a thymine in a polynucleotide sequence is a nucleotide substitution.

"Conservative substitution", when used with reference to a polypeptide, refers to a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

As used herein, "vector" refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "% sequence identity" and "percent identity" refers to sequence identity between a nucleotide sequence and a reference nucleotide sequence or between an amino acid sequence and a reference amino acid sequence. Sequence identity can be determined by comparing a position in each sequence which may be aligned for purposes of comparison. When a position in the compared sequence is occupied by the same base or amino acid, then the molecules are identical at that position. A degree of identity between nucleotide or amino acid sequences is a function of the number of identical or matching nucleotides or amino acids at positions shared by the nucleotide or amino acid sequences, respectively. Various alignment algorithms and/or programs may be used to calculate the identity between two sequences, including FASTA or BLAST which are available as a part of the GCG sequence analysis package (University of Wisconsin, Madison, Wis.), and can be used with default settings.

As used herein, the term "about" means plus or minus 10% of the numerical value of the number with which it is being used.

Where a numerical limit or range is stated herein, the endpoints are included. Also, all values and sub ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components, or groups thereof.

Having generally described this invention, a further understanding can be obtained by reference to certain specific examples, which are provided herein for purposes of illustration only, and are not intended to be limiting unless otherwise specified.

### Examples

### Example 1: Reducing equivalents from methanol via linear dissimilation pathways

Methanol is a highly reduced compound with a degree of reduction per atom of carbon of 6. Compared to glucose, the most common biotechnological feedstock, this is 50% higher. This means that methanol provides more electrons per carbon. If these additional electrons are used in product synthesis, it can lead to improved yields. A group of organisms called methylotrophs is capable of utilizing methanol as the sole source of carbon and energy.

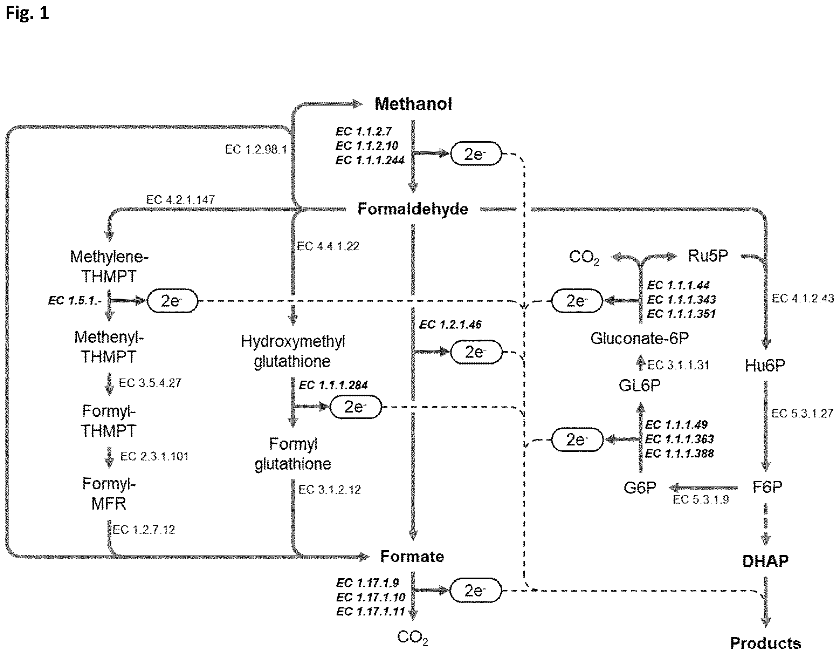

Several pathways for transfering electrons from methanol onto electron carriers such as NAD+ or NADP+ exist (Figure 1). The first enyzmatic step in methanol assimilation, dehydrogenation into formaldehyde, can release 2 electrons in the form of 1 NADH molecule per molecule of methanol.

This reaction is catalysed by NAD-dependent methanol dehydrogenase (EC 1.1.1.244, SEQ ID 1,2,3). However, in many methylotrophs, this step is catalyzed by a pyrroloquinoline quinone (PQQ) dependent dehydrogenase (EC 1.1.2.7 - SEQ ID 4,5,6, 1.1.2.10 - SEQ ID 7), which produces reduced PQQ. This cannot be used for product synthesis, instead it can only enter the respiratory chain.

The resulting formaldehyde can then enter the central metabolism of methylotrophs through different pathways to form biomass and other products, or it can enter various dissimilation pathways that ultimately produce CO₂ and reduced electron carriers (Figure 1).

Formaldehyde can be directly converted into formate by the enzyme formaldehyde dehydrogenase (EC 1.2.1.46 - SEQ ID 8, 9, 10, 11), which produces NADH, respectively. Alternatively, formaldehyde can be attached to glutathione to form S-(Hydroxymethyl)glutathione by the enzyme S-(hydroxymethyl)glutathione synthase (EC 4.4.1.22 - SEQ ID 12,13,14,15). S-(Hydroxymethyl)glutathione is the substrate of the enzyme S-(hydroxymethyl)glutathione dehydrogenase (EC 1.1.1.284 - SEQ ID 16,17,18,19,20,21), which converts it into S-formyl-glutathione, producing NADH or NADPH. S-formyl-glutathione is then cleaved into formate and glutathione by S-formylglutathione hydrolase (EC 3.1.2.12 - SEQ ID 22,23,24,25,26,27). Formaldehyde can also enter the tetrahydromethanopterin (THMPT) pathway. As the first step, formaldehyde reacts with THMPT to form methylene-THMPT. Methylene-THMPT is the substrate of methylenetetrahydromethanopterin dehydrogenase (EC 1.5.1.- SEQ ID 28,29,30), which produces NADPH or and methenyl-THMPT. Methenyl-THMPT is ultimately converted into formate in several additional steps, though no electrons are released here. Finally, formaldehyde dismutase (EC 1.2.98.1 - SEQ ID 31,32,33) can react formaldehyde with water, producing formate and methanol. This does not produce reduced electron carriers directly, but the resulting methanol can be converted into formaldehyde again, producing NADH (Figure 1).

Regardless of the pathway used to produce formate from methanol, 4 electrons per molecule can be released up to this point. Formate can then be converted to CO₂ by the NAD-, NADP- or Fd-dependent formate dehydrogenase (EC 1.17.1.9 - SEQ ID 34,35,36, EC 1.17.1.10 - SEQ ID 38,39,40, 1.17.1.11 - SEQ ID 37) releasing 2 additional electrons. These NADH/NADPH/Fd_{red} can either be used for ATP formation or for product synthesis in reactions that require reducing equivalents.

### Example 2: Reducing equivalents from methanol via oxidative RuMP cycle

In methylotrophic organisms expressing the Ribulose Mono Phosphate (RuMP) assimilation pathway, formaldehyde enters the metabolism by reacting to Ribulose-5-phosphate (Ru5P) to form Hexulose-6-phosphate (Hu6P) in a reaction catalysed by the 3-hexulose-6-phosphate synthase (HPS) enzyme (EC 4.1.2.43 - SEQ ID 41,42,43). Hu6P is converted into Fructose-6-phosphate (F6P) by 3-hexulose-6-phosphate isomerase (HPI) (EC 5.3.1.27 - SEQ ID 44,45,46). F6P is a branching point in the metabolism and can enter multiple downstream pathways. It can be converted into Glucose-6-phosphate by glucose-6-phosphate isomerase (GPI) (EC 5.3.1.9 - SEQ ID 47,48,49,50), and then into glucono-1,5-lactone-6P (GL6P) by NAD-dependent (EC 1.1.1.388 - SEQ ID 51) or NADP-dependent (EC 1.1.1.363 - SEQ ID52,53,54,55, EC 1.1.1.49 - SEQ ID 56,57,58,59,60,61) glucose-6-phosphate dehydrogenase. This step releases 2 electrons in the form of NADH or NADPH, respectively. GL6P is converted into Gluconate-6P by 6-phosphogluconolactonase (EC 3.1.1.31 - SEQ ID 62,63,64,65,66,67). Gluconate-6P represents another branching point in the metabolism. It can be dissimilated into CO₂ and NADH or NADPH in the final formaldehyde dissimilation pathway or be assimilated into biomass and products (Figure 1).

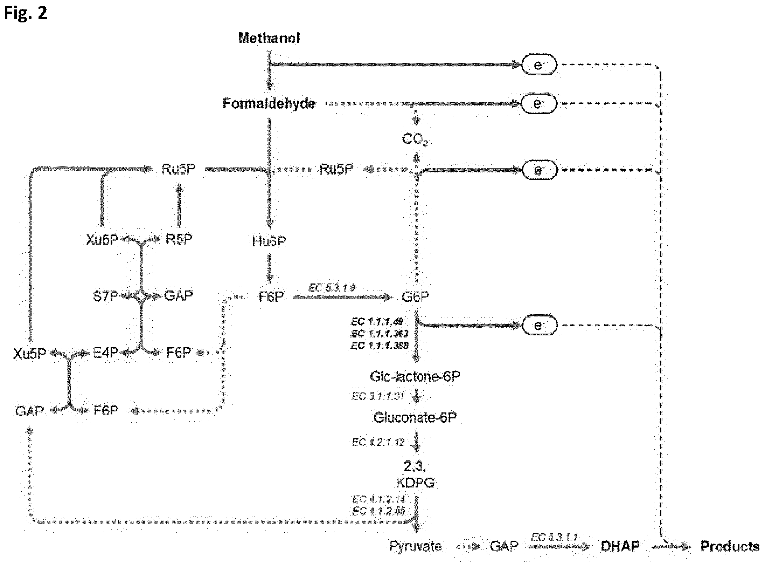

If dissimilated, it is converted into Ribulose-5-Phosphate (Ru5P) and CO₂ by NAD-dependent (EC 1.1.1.343 - SEQ ID 68,60,70) or NADP-dependent (EC 1.1.1.44 - SEQ ID 71,72,73,74,75,76, EC 1.1.1.351 - SEQ ID 77,78,79) phosphogluconate dehydrogenase. This releases an additional 2 electrons in the form of NADH or NADPH, respectively. The Ru5P re-enters the assimilation cycle and reacts with formaldehyde (Figure 1, Figure 2)).

If assimilated, Gluconate-6P is first converted into 2-Keto-3-deoxy-6-phosphogluconate (2,3-KDPG) by 6-phosphogluconate dehydratase (EDD) (EC 4.2.1.12 - SEQ ID 80,81,82,83,84) and then into Pyruvate and D-Glyceraldehyde 3-phosphate (GAP) by 2,3-KDPG aldolase (EDA) (EC 4.1.2.14 - SEQ ID 85,86,87,88,89, and EC 4.1.2.55 - SEQ ID 90,91). GAP re-enters the assimilation cycle to regenerate Ru5P and pyruvate enters the TCA cycle and product synthesis. This particular configuration is termed the EDA-variant of the RuMP cycle (Figure 2).

### Example 3: In-silico metabolic engineering of DHAP synthesis

GAP can be converted into DHAP by triosephosphate isomerase (TPI) (EC 5.3.1.1 - SEQ ID 92,93,94,95,96,97) and vice-versa. In EDA-RuMP methylotrophs, GAP produced by EDA-mediated cleavage of 2,3-KDPG must re-enter the RuMP assimilation cycle to prevent Ru5P depletion. The only way of obtaining GAP that can be used for product synthesis is through gluconeogenesis, where pyruvate produced by EDA is ultimately converted into GAP. Producing 1 molecule of GAP in this way requires the additional investment of 2 ATP and 1 NADH or NADPH molecules, which reduces product yield.

Alternatively, a phosphofructokinase (pfk) enyzme can be added the the system. Pfk catalyses phosphorilation of F6P into fructose-1,6-bisphosphate (1,6-FBP), consuming an ATP, ADP or pyrophosphate (EC 2.7.1.11 - SEQ ID 98,99,100,101,102,103, EC 2.7.1.146 - SEQ ID 104,105,106, EC 2.7.1.90 - SEQ ID 107,108,109, respectively). The resulting 1,6-FBP can then be cleaved into GAP and DHAP by fructose-bisphosphate aldolase (EC 4.1.2.13 - SEQ ID 110,111,112,113,114,115). The GAP must re-enter the assimilation cycle to prevent Ru5P delpetion, but the DHAP can be used for product synthesis. This pathway requires no additional input of ATP or NADH/NADPH and is therefore significantly more energy efficient. In this case, deletion or inactivation of TPI (EC 5.3.1.1 - SEQ ID 92,93,94,95,96,97) would enforce DHAP accumulation, provided no alternative sink is present in the metabolism. In such a case, EDA-derived pyruvate would provide the carbon required for cellular components while DHAP would be used solely for chemical production. Control of carbon flux through EDA would dictate the ratio of carbon dedicated to cell growth and product synthesis.

Implementing this pathway in a methylotrophic organism expressing one or more of the methanol dissimilation pathways described in Examples 1 and 2 can lead to significantly higher yields compared to implementing it in a glucose-consuming organism. Synthesis of DHAP-derived requires reducing equivalents. In glucose metabolism, no reduced cofactors are produced up to the point of 1,6-FBP cleavage into GAP and DHAP. The only way to obtain reducing equivalents in such a case is by converting the GAP moiety into pyruvate through glycolysis. This means that 50% or more of the feedstock carbon cannot be used in product formation. If implemented in a methylotroph where methanol dissimilation is used to produce reducing equivalents, only 14% of carbon must be dissimilated to CO₂ in order to produce sufficient reducing equivalents. This means the practical maximum of such products in a methylotroph is ^{~}70% higher compared to glucose. For simplicity, these calculations are based on products that require reducign equivalents in a single enzymatic step (e. g. Glycerol). When they are required in multiple steps (e. g. 1,2-PDO), the principle remains the same, but more reduced carbon species are oxidised to CO₂ in order to source reducing equivalents.

### Example 4: Metabolic engineering of glycerol synthesis

DHAP is the precursor for many industrially relevant 3-carbon compounds with Formula I:

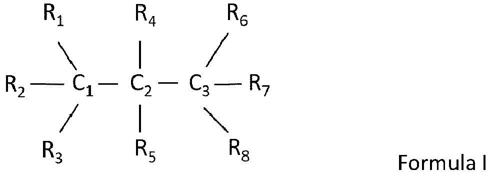

Glycerol is an example of such compounds. It is synthesised from DHAP in two steps, the first catalysed by the NADH- or NADPH-dependent enzyme glycerol-phosphate dehydrogenase (EC 1.1.1.8 - SEQ ID 116,117,118,119,120,121, EC 1.1.1.94 - SEQ ID 122,123,124,125, respectively). The first reaction produces glycerol phosphate, which is then dephosphorylated by glycerol phosphatase (EC 3.1.3.21 - SEQ ID 126,127,128,129,130,131). Those skilled in the art can match the reduced cofactor required by this step with the ones released during methanol dissimilation by expressing enzymes with different cofactor specificities in the host methylotrophic strain.

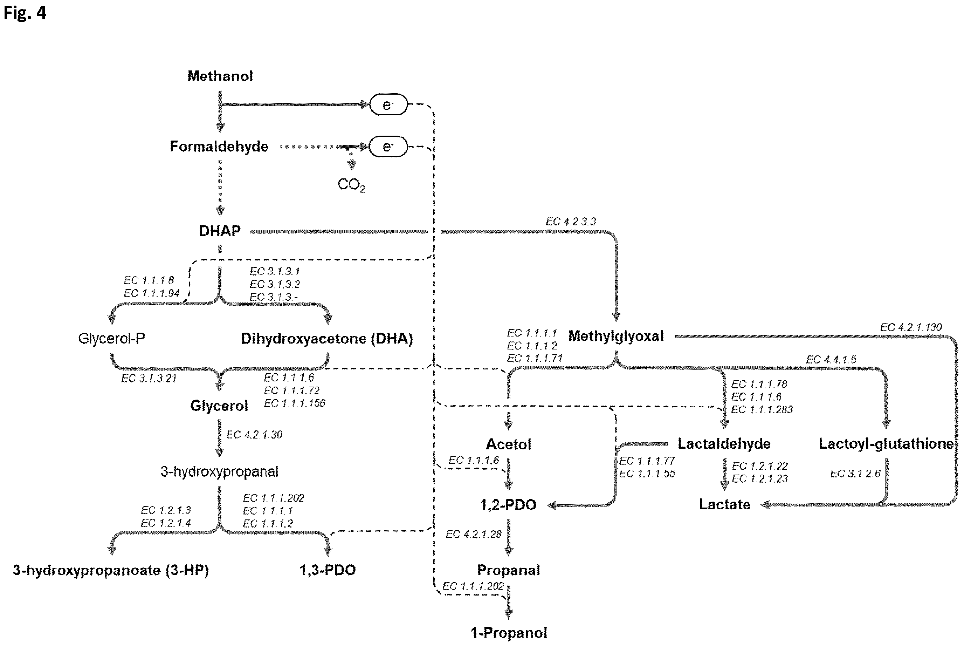

Dihydroxyacetone (also glycerone) can be synthesised from DHAP by dephosphorylation. The reaction is carried out by alkaline and acidic phosphatases (EC 3.1.3.1 - SEQ ID 211,212, EC 3.1.3.2 - SEQ ID 213,214), such as the DHAP-specific hdpA from *Corynebacterium glutamicum* (strain R) (EC 3.1.3.- - SEQ ID 132). The resulting dihydroxyacetone can then be converted into dihydroxyacetone by a glycerol dehydrogenase enzyme in a reaction that consumes NADPH (EC 1.1.1.72 - SEQ ID 135,136, EC 1.1.1.156 - SEQ ID 137,138,139,140) or either NADH/NADPH (EC 1.1.1.6 - SEQ ID 133,134), such as those from *Citrobacter freundii* and *Clostridium butyricum* (SEQ 133, 134). (Figure 4)

### Example 5: Metabolic engineering of Hydroxyacetone (Acetol) synthesis

DHAP can be converted into Methylglyoxal (also 2-Oxopropanal or Pyruvaldehyde) through a reaction catalysed by methylglyoxal synthase (EC 4.2.3.3 - SEQ ID 141,142,143,144,145,146). Methylglyoxal is the precursor for several industrally relevant 3-carbon compounds, for example Hydroxyacetone (also Acetol). The conversion of methylglyoxal into acetol is catalysed by methylglyoxal-specific alcohol dehydrogenases that can be NADH-dependent (EC 1.1.1.1 - SEQ ID 147) or NADPH-dependent (EC 1.1.1.2 - SEQ ID 148,149,150,151,152,153, EC 1.1.1.71 - SEQ ID 154,155,156). Those skilled in the art can match the reduced cofactor required by this step with the ones released during methanol dissimilation by expressing enzymes with different cofactor specificities in the host methylotrophic strain.

### Example 6: Metabolic engineering of Lactaldehyde (2-hydroxypropanal) synthesis

DHAP can be converted into Methylglyoxal (also 2-Oxopropanal or Pyruvaldehyde) through a reaction catalysed by methylglyoxal synthase (EC 4.2.3.3 - SEQ ID 141,142,143,144,145,146). Methylglyoxal is the precursor for several industrally relevant 3-carbon compounds, for example Lactaldehyde (also 2-hydroxypropanal). Methylglyoxal can be converted into lactaldehyde by NADH or NADPH-dependent lactaldehyde dehydrogenases (EC 1.1.1.78 - SEQ ID 157 and EC 1.1.1.283 - SEQ ID 158,159,160,161, respectively). Alternatively, the conversion can be carried out by side activity of some non-specific NADH-dependent glycerol dehydrogenases (EC 1.1.1.6 - SEQ ID 162,163,164), such as those from *Klebsiella aerogenes* or *Schizosaccharomyces pombe,* in a reaction that consumes NADH. Those skilled in the art can match the reduced cofactor required by this step with the ones released during methanol dissimilation by expressing enzymes with different cofactor specificities in the host methylotrophic strain. (Figure 4)

### Example 7: Metabolic engineering of lactic acid synthesis

Lactaldehyde (detailed in Example 6) can also be converted into lactic acid. This conversion is carried out by the enzyme lactaldehyde dehydrogenase (EC 1.2.1.22 - SEQ ID 165,166,167,168, EC 1.2.1.23 - SEQ ID 169) in a reaction that consumes NADH or NADPH. Those skilled in the art can match the requirement for reduced cofactors with the appropriate enzymes in the described methanol dissimilation pathways.

Alternatively, lactic acid can be synthesised from methylglyoxal in a single step, catalysed by lactate-dehydratase (EC 4.2.1.130 - SEQ ID 170,171,172,173), such as those from *Candida albicans* and *Schizosaccharomyces pombe.* Methylglyoxal can also be converted into lactic acid in two steps, via lactoyl-glutathione. The first step is catalysed by lactoylglutathione lyase (EC 4.4.1.5 - SEQ ID 174,175,176,177,178,179) and the second by hydroxyacylglutathione hydrolase (EC 3.1.2.6 - SEQ ID 180,181,182,183,184,185). (Figure 4)

### Example 8: Metabolic engineering of Propane-1,2-diol synthesis

Propane-1,2-diol (also 1,2-PDO) can also be produced from DHAP via two different pathways. Both pathways first require synthesis of methylgyoxal by methylglyoxal synthase (EC 4.2.3.3 - SEQ ID 141,142,143,144,145,146), as detailed in example 6. In the first pathway, methylglyoxal is first converted into lactaldehyde by NADH or NADPH-dependent lactaldehyde dehydrogenases (EC 1.1.1.78 - SEQ ID 157, EC 1.1.1.283 - SEQ ID 158,159,160,161, respectively) or NADH-dependent glycerol dehydrogenase (EC 1.1.1.6 - SEQ ID 133,134). Lactaldehyde can then be converted into 1,2-PDO by lactaldehyde reductase enzymes (EC 1.1.1.77 - SEQ ID 186,187,188,189) in a reaction that consumes NADH or NADPH. Alternatively, an NADPH-dependent lactaldehyde reductase (EC 1.1.1.55 - SEQ ID 190,191) can also be used.

The second pathay involves synthesis of acetol from methylglyoxal by be NADH-dependent (EC 1.1.1.1 - SEQ ID 147) or NADPH-dependent (EC 1.1.1.2 - SEQ ID 148,149,150,151,152,153, EC 1.1.1.71 - SEQ ID 154,155,156) alcohol dehydrogenases specific for methylglyoxal, as described in Example 5. Acetol can then be converted into 1,2-PDO by side activity of a glycerol dehydrogenase (EC 1.1.1.6 - SEQ ID 192,193) that accepts acetol as a substrate, such as that from *Escherichia coli.* This reaction consumes NADH or NADPH.

If either pathway is expressed in a methylotrophic organism, those skilled in the art can match the reduced cofactor requirements with the enzymes used by methanol dissimilation pathways. (Figure 4)

### Example 9: Metabolic engineering of 3-hydroxypropanal synthesis

3-hydroxypropanal can be synthesised from DHAP via glycerol, which is described in Example 4. Glycerol is synthesised from DHAP in two steps, the first catalysed by the NADH- or NADPH-dependent enzyme glycerol-phosphate dehydrogenase (EC 1.1.1.8 - SEQ ID 116,117,118,119,120,121, EC 1.1.1.94 - SEQ ID 122,123,124,125, respectively This reaction produces glycerol phosphate, which is then dephosphorylated by glycerol phosphatase (EC 3.1.3.21 - SEQ ID 126,127,128,129,130,131). The resulting glycerol can be converted into 3-hydroxypropanal by glycerol dehydratase (EC 4.2.1.30- SEQ ID 194,195,196). When the pathway expressed in a methylotrophic organism, those skilled in the art can match the reduced cofactor requirements with the enzymes used by methanol dissimilation pathways. (Figure 4)

### Example 10: Metabolic engineering of Propane-1,3-diol synthesis

Propane-1,3-diol (or 1,3-PDO) can be synthesised from DHAP via glycerol, which is described in Example 4. Glycerol is synthesised from DHAP in two steps, the first catalysed by the NADH- or NADPH-dependent enzyme glycerol-phosphate dehydrogenase (EC 1.1.1.8 - SEQ ID 116,117,118,119,120,121, EC 1.1.1.94 - SEQ ID 122,123,124,125, respectively This reaction produces glycerol phosphate, which is then dephosphorylated by glycerol phosphatase (EC 3.1.3.21 - SEQ ID 126,127,128,129,130,131). The resulting glycerol can be converted into 3-hydroxypropanal by glycerol dehydratase (EC 4.2.1.30 - SEQ ID 194,195,196) as detailed in example 10. 3-hydroxypropanal is then converted into 1,3-PDO by 1,3-PDO dehydrogenase (EC 1.1.1.202 - SEQ ID 209, 210) in a reaction that consumes NADH or NADPH. Alternatively, NADH- or NADPH-dependent alcohol dehydrogenases (EC 1.1.1.1 - SEQ ID 147 and EC 1.1.1.2 - SEQ ID 148,149,150,151,152,153, respectively) with broad substrate specificity can also be used. When the pathway expressed in a methylotrophic organism, those skilled in the art can match the reduced cofactor requirements with the enzymes used by methanol dissimilation pathways. (Figure 4)

### Example 11: Metabolic engineering of 3-hydroxypropionic acid synthesis

3-hydroxypropionic acid (or 3-HPO) can be synthesised from DHAP via glycerol, which is described in Example 4. Glycerol is synthesised from DHAP in two steps, the first catalysed by the NADH- or NADPH-dependent enzyme glycerol-phosphate dehydrogenase (EC 1.1.1.8 - SEQ ID 116,117,118,119,120,121, EC 1.1.1.94 - SEQ ID 122,123,124,125, respectively). This reaction produces glycerol phosphate, which is then dephosphorylated by glycerol phosphatase (EC 3.1.3.21 - SEQ ID 126,127,128,129,130,131). The resulting glycerol can be converted into 3-hydroxypropanal by glycerol dehydratase (EC 4.2.1.30 - SEQ ID 194,195,196) as detailed in example 10. 3-hydroxypropanal is then converted into 3-HP by an NADH-dependent aldehyde dehydrogenase (EC 1.2.1.4 - SEQ ID 197,198) specific for 3-hydroxypropanal, such as that from *Klebsiella pneumoniae* or *Escherichia coli* (SEQ ID 197, 198) or an NADPH-specific aldehyde dehydrogenase (EC 1.2.1.3 - SEQ ID 199,200,201,202,203,204). When the pathway expressed in a methylotrophic organism, those skilled in the art can match the reduced cofactor requirements with the enzymes used by methanol dissimilation pathways. (Figure 4)

### Example 12: Metabolic engineering of propanal and 1-propanol synthesis

Propanal can be syntehsised from DHAP via 1,2-PDO, detailed in Example 8. Propane-1,2-diol (also 1,2-PDO) can also be produced from DHAP via two different pathways. Both pathways first require synthesis of methylgyoxal by methylglyoxal synthase (EC 4.2.3.3 - SEQ ID 141,142,143,144,145,146), as detailed in example 6. In the first pathway, methylglyoxal is first converted into lactaldehyde by NADH or NADPH-dependent lactaldehyde dehydrogenases (EC 1.1.1.78 - SEQ ID 157 - SEQ ID 157, EC 1.1.1.283 - SEQ ID 158,159,160,161, respectively) or NADH-dependent glycerol dehydrogenase (EC 1.1.1.6 - SEQ ID 133,134). Lactaldehyde can then be converted into 1,2-PDO by lactaldehyde reductase enzymes (EC 1.1.1.77 - SEQ ID 186,187,188,189) in a reaction that consumes NADH or NADPH. Alternatively, an NADPH-dependent lactaldehyde reductase (EC 1.1.1.55 - SEQ ID 190,191) can also be used.

The second pathay involves synthesis of acetol from methylglyoxal by be NADH-dependent (EC 1.1.1.1 - SEQ ID 147) or NADPH-dependent (EC 1.1.1.2 - SEQ ID 148,149,150,151,152,153, EC 1.1.1.71 - SEQ ID 154,155,156) alcohol dehydrogenases specific for methylglyoxal, as described in Example 5. Acetol can then be converted into 1,2-PDO by side activity of a glycerol dehydrogenase (EC 1.1.1.6 - SEQ ID 192,193) that accepts acetol as a substrate, such as that from *Escherichia coli.* This reaction consumes NADH or NADPH.

1,2-PDO can be converted into propanal by propanediol dehydratase (EC 4.2.1.28 - SEQ ID 205,206,207,208). 1-propanol can be synthesised from propanal by 1-propanol dehydrogenase (EC 1.1.1.202 - SEQ ID 209, 210) in a reaction that consumes NADH or NADPH. (Figure 4)

### Example 13: Expression of recombinant genes in Methylobacillus

Different combinations of GPP and GPD genes (SEQ_ID 116, 117, 122, 123, 130, 131) were cloned into an expression vector carrying a broad host range origin of replication under the control of an IPTG-inducible lacO/trc promotor. Cloning was done using a NEB Hi-Fi cloning kit according to manufacturer instructions. The prepared plasmids were checked by sequencing and transformed into *M. flagellatus* via electroporation to create strains *Methylobacillus* OCB592, OCB593, OCB594, OCB595 and OCB596.

| **Strain** | **GPD** | **GPP** | **Glycerol [g/L]** |
|---|---|---|---|
| **OCB480** | / | / | 0 |
| **OCB592** | 122 | 130 | 0.14 |
| **OCB593** | / | 131 | 0.28 |
| **OCB594** | 116 | 130 | 0.49 |
| **OCB595** | 123 | 131 | 0.17 |
| **OCB596** | 117 | 131 | 0.53 |

### Example 14: Glycerol production in shake flasks

To test glycerol production, transformants were cultivated in 250 mL shake flasks in 50 mL of a mineral medium containing 1% (V/V) methanol, KH2PO4, Na2HPO4, MgSO4, NH4SO4, and trace elements. The shake flasks were inoculated with 2.5 mL liquid overnight culture. After 8 h of growth, 0.5 g/L IPTG was added to induce gene expression. The cultures were sampled after 24 h of growth and cells were removed by centrifugation. Glycerol content in the supernatant was measured by HPLC.

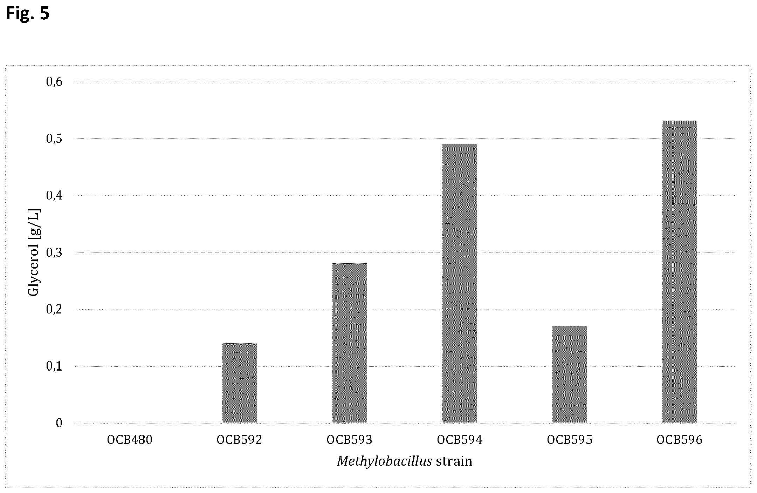

The strain OCB 596, expressing the NAD-dependent GPD from *Schizosaccharomyces pombe* and GPP2 from *Saccharomyces cerevisiae* produced the highest titer of glycerol at 0.53 g/L. Under the same conditions, the control strain that did not express heterologous GPP or GPD did not produce detectable amounts of glycerol (Figure 5).

## Claims

1. A genetically engineered microorganism which is methylotrophic and can generate reducing power by methanol dissimilation, and said microorganism being modified to enable the synthesis of at least one DHAP-derived compound.

2. The genetically engineered microorganism according to claim 1, which can metabolize methanol as the main carbon source and main electron donor via the methanol dissimilation pathway.

3. The genetically engineered microorganism according to any one of claims 1-2, wherein reducing equivalents are obtained via NAD- and or NADP dependent glucose-6-phosphate 1-dehydrogenase and NAD and/or NADP- dependent 6-phosphogluconate dehydrogenase.

4. The genetically engineered microorganism according to any one of claims 1-2, wherein reducing equivalents are obtained via formaldehyde dehydrogenase and/or formate dehydrogenase.

5. The genetically engineered microorganism according to any one of claims 1-2, wherein reducing equivalents are obtained via the Methylene-THMPT pathway.

6. The genetically engineered microorganism according to any one of claims 1-5, comprising the ribulose monophosphate (RuMP) methanol assimilation pathway.

7. The genetically engineered microorganism according to claim 6, wherein said genetically engineered microorganism has the EDA variant of RuMP.

8. The genetically engineered microorganism according to any one of claims 1-7, wherein said DHAP-derived compound has three carbons.

9. The genetically engineered microorganism according to any one of claims 1-8, wherein said DHAP-derived compound has the structure of Formula I : 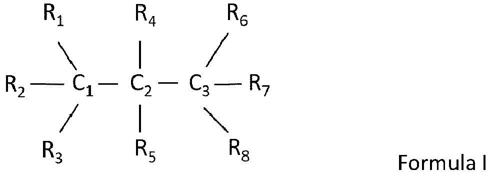
wherein R₁, R₂, R₃, R₄, R₅, R₆ and R₇ are independently selected from hydrogen, hydroxyl, and pairwise on each of carbons C₁ to C₃ may form an oxo group, and
wherein R₈ is selected from hydrogen, hydroxyl and phosphate.

10. The genetically engineered microorganism according to any one of claims 1-9, wherein said DHAP-derived compound is selected from the group consisting of glycerol, 1,3-propanediol, 1,2-propanediol, methylglyoxal (MGO), 2-hydroxypropanal (lactaldehyde), 3-hydroxypropanal (reuterin), hydroxyacetone (acetol), dihydroxyacetone (glycerone), 3-hydroxypropionate, lactate, malonate, glycerone, acetone and dihydroxyacetone phosphate.

11. The genetically engineered microorganism according to any one of claims 1-10, wherein there is absence of expression of triosephosphate isomerase (TPI).

12. The genetically engineered microorganism according to any of claims 1-11, wherein the expression of phosphogluconate dehydratase (EDD) is lowered and the expression of phosphofructokinase (pfk) is increased.

13. The genetically engineered microorganism according to any of claims 11-12, which can express a heterologous phosphofructokinase.

14. The genetically engineered microorganism according to any of claims 1-13, wherein said microorganism is a *Methylobaccilus sp.*

15. A method for the production of a biochemical compound derived from DHAP comprising cultivating a microorganism according to any one of claims 1-14 under suitable culture conditions in a culture medium comprising a reduced one-carbon compound, such as methanol, or a multi-carbon compound that contains no carbon-carbon bonds, such as dimethylamine or methylamine.
